# EUROPÄISCHE PATENTSCHRIFT

(11) **EP 2 803 354 B1**
(45) Veröffentlichungstag und Bekanntmachung des Hinweises auf die Patenterteilung: **04.03.2015**
(21) Anmeldenummer: 13167597.7
(22) Anmeldetag: 14.05.2013
(51) Int. Cl.: A61K 31/133, A61K 31/78, A61K 45/06, A61P 31/22, A61P 43/00

(54) **Kombination aus Polyacrylsäure und 2-Amino-2-methylpropanol zur Verwendung in der Behandlung von Herpesinfektionen**
Combination of polyacrylic acid and 2-amino-2-methylpropanol for use in the treatment of Herpes infections
Combinaison de l'acide polyacrylique et du 2-amino-2-methylpropanol pour l'utilisation dans le traitement des infections de l'Herpès

(43) Veröffentlichungstag der Anmeldung: 19.11.2014
(73) Patentinhaber: NajöPharm GmbH i.G., 22087 Hamburg (DE)
(72) Erfinder: Heiser, Nadine, 22087 Hamburg (DE)
(74) Vertreter: Glawe, Delfs, Moll

(56) Entgegenhaltungen:
- WO-A1-95/11663
- WO-A1-2011/015628
- WO-A1-2012/004669
- US-A1- 2013 072 575
- DATABASE MEDLINE [Online] US NATIONAL LIBRARY OF MEDICINE (NLM), BETHESDA, MD, US April 2005 OSTUNI P ET AL: '[Efficacy of Carbopol 974P (Siccafluid) in the treatment of severe to moderate keratoconjunctivitis sicca in patients with primary Sjögren's syndrome not responding to standard treatment with artificial tears].' Database accession no. NLM15983636 & REUMATISMO 2005 APR-JUN, vol. 57, no. 2, April 2005 (2005-04), pages 119-124, ISSN: 0048-7449

## Beschreibung

Die vorliegende Erfindung betrifft eine Zusammensetzung mit Aktivität gegen Viren der Herpesfamilie, und ein Verfahren zur Verwendung dieser Zusammensetzung zur Behandlung bei der prophylaktischen und/oder therapeutischen Behandlung von Herpes-Infektionen.

### Hintergrund der Erfindung

Die Familie der Herpesviren umfasst die Herpes-simplex-Viren vom Typ HSV 1 und HSV 2 sowie das Varicella-Zoster-Virus (VZV). Die Primärinfektion mit dem HSV verursacht typischerweise Läsionen am Ort der Infektion, die in der Regel innerhalb von 2-3 Wochen abheilen, wonach das Virus zu den Ganglien wandert, in denen es in einer Latenzphase bis zur Aktivierung verbleibt. Die durch einen Reiz wie beispielsweise Stress, Erkältung, Fieber, Immunschwäche, übermäßige Sonnenbestrahlung, unzulängliche Ernährung, Menstruation etc. ausgelöste Aktivierung führt zu sekundären Ausbrüchen (rezidivierende Infektion), die sich bei HSV 1 typischerweise an den Lippen als Herpes labialis manifestiert und bei HSV 2 typischerweise Läsionen an den Genitalien oder in deren Umgebungsbereich verursacht. Infektionen mit den HSV Serotypen sind weit verbreitet. So tragen schätzungsweise rund 90% der Menschen HSV 1 in sich und viele der Virusträger erleben mindestens einmal im Leben die schmerzhaften Fieberbläschen an den Lippen [W. Hellenbrand *et al*.: Seroprevalence of herpes simplex virus type 1 (HSV-1) and type 2 (HSV-2) in former East and West Germany, 1997-1998; Eur. J. Clin. Microbiol. Infect. Dis. (2005) 24(2): S. 131-135].

Die Heilung vorgenannter Herpes-Infektionen ist nach aktuellem Wissenstand nicht möglich. Antivirale Therapien verringern die Symptome bei sekundären Ausbrüchen bei den Patienten in unbefriedigendem Maße. Besonders betroffen sind davon "austherapierte" Patienten, bei denen sich im Zuge der Behandlung mit beispielsweise dem derzeit gebräuchlichsten Virustatika Aciclovir [2-Amino-9-(2-hydroxyethoxy)methyl-1,9-dihydro-6H-purin-6-on, im Handel unter Zovirax® u.a.] aciclovirresistente Herpesstämme isolieren ließen.

Es besteht folglich Bedarf an neuen Stoffen und Verfahren zur Behandlung von Herpes-Infektionen.

### Beschreibung der Erfindung

Aufgabe der vorliegenden Erfindung ist es, ein wirksames Mittel als Alternative oder Ergänzung zu aus dem Stand der Technik bekannten Behandlungsmöglichkeiten von Herpes-Infektion zur Verfügung zu stellen.

Gelöst wird diese Aufgabe durch eine Zusammensetzung umfassend eine Wirkstoffkombination aus Polyacrylsäure und 2-Amino-2-methylpropanol zur Verwendung als Medikament, insbesondere zur prophylaktischen und/oder therapeutischen Behandlung von Herpes-Infektionen gemäß den Ansprüchen 1 und 2. Vorteilhafte Ausführungsformen der Erfindung sind in den Unteransprüchen offenbart. Überraschend hat sich gezeigt, dass die erfindungsgemäße Zusammensetzung die Dauer einer Herpes-Infektion verringern kann und ebenfalls zur Prophylaxe zur Unterdrückung von rezidivierendem Herpes geeignet ist.

Zunächst seien einige im Rahmen der Erfindung verwendete Begriffe erläutert.

Die erfindungsgemäße Zusammensetzung wird zur Behandlung eines Objekts oder Individuums eingesetzt, das an einer Herpes-Infektion, insbesondere *Herpes labialis* (Lippenherpes), *Gingivostomatitis herpetica* (Mundfäule) oder *Herpes simplex genita lis* (Genitalherpes), leidet. Die zu behandelnde Herpes-Infektion kann durch den Herpes-simplex-Virus vom Typ 1 oder 2 oder Varicella-Zoster-Virus (Herpes zoster) ausgelöst sein. Der Begriff "Objekt" umfasst lebende Tiere und Menschen. Der Zweck dieser Behandlung ist die zumindest teilweise Linderung der Symptome der Herpes-Infektion und insbesondere die Verkürzung der rezidiven Phasen, wie sie nachfolgend für den Lippenherpes (Herpes labialis) kurz zusammengefasst sind:
In der Prodromalphase (Tag 0-1) treten üblicherweise die folgenden Symptome auf: Schmerzen, Kribbeln, Brennen, Spannungsgefühl und Jucken, die zeitlich mit der frühen Phase der Virusreplikation in der Haut korrelieren, die hauptsächlich in den ersten 48h stattfindet. In der sich anschließenden Papelphase (Tag 2-3) rötet sich zunächst die Haut, bevor am Lippenrand mehr oder weniger dicke gerötete und für den Patienten schmerzhafte Pappeln am Lippenrand bilden, die sich in der Vesikelphase zu flüssigkeitsgefüllten Bläschen mit einem Durchmesser zwischen 2-5 mm entwickeln und anschließend in der Ulzerationsphase (Tag 4) aufbrechen und sich zu schmerzhaft nässenden Wunden verbinden. Zum Ende der Infektion folgt dann die Verkrustungs- (Tag 5-8) und Abheilungsphase (Tag 9-14) in der sich unter der Kruste neue Haut bildet. Unbehandelt ist der Lippenherpes meist nach 2 Wochen abgeheilt. Eine gefürchtete Variante des Lippenherpes ist eine Beteiligung des Auges, insbesondere der Hornhaut, als Folge derer Hornhauttrübungen und Hornhautnarben mit einhergehenden Sehstörungen bis hin zur Erblindung auftreten können.

Die vorliegende Erfindung bezieht sich auch auf die Behandlung eines an einer Herpes-Infektion leidenden Objekts, wobei dem Objekt eine therapeutisch wirksame Menge der erfindungsgemäßen Zusammensetzung verabreicht wird.

Der Begriff "Behandlung" bedeutet im Sinne der vorliegenden Erfindung das ganze oder teilweise Erreichen der nachfolgend genannten Ergebnisse: Ganze oder teilweise Reduktion des Krankheitsbildes; Verbesserung mindestens eines der klinischen Symptome oder mit der Krankheit assoziierten Indikatoren; Verzögerung, Unterdrückung oder Schutz vor dem Fortschreiten der Krankheit; oder ganze oder teilweise Verzögerung, Unterdrückung oder Schutz vor dem Ausbrechen oder Entstehung der Krankheit. Das zu behandelnde Objekt ist ein Mensch oder Tier, vorzugsweise ein Säugetier.

Der Begriff "Zusammensetzung umfassend Polyacrylsäure und 2-Amino-2-methylpropanol" schließt die Zusammensetzung als Kombinationspräparat aus den beiden genannten therapeutisch aktiven Komponenten ohne weitere therapeutisch aktive Komponenten ein. Die erfindungsgemäße Zusammensetzung kann allein oder in Kombination mit mindestens einer anderen therapeutischen Substanz zur Verringerung eines oder mehrerer Symptome der Herpes-Infektion verabreicht werden. Die Verabreichung der erfindungsgemäßen Zusammensetzung kann gleichzeitig mit der anderen therapeutischen Substanz (z.B. ätherische Öle, wie beispielsweise aus Melisse, Pfefferminze; Braunelle, Rosmarin, Salbei oder Thymian, Teebaum und Eukalyptus), welche Bestandteil derselben Zusammensetzung sein kann oder in einer anderen Zusammensetzung bereitgestellt wird, verabreicht werden. Alternativ kann die erfindungsgemäße Zusammensetzung vor oder nach der Verabreichung der anderen therapeutischen Substanz verabreicht werden. Die erfindungsgemäße Zusammensetzung kann über den gleichen oder einen anderen Verabreichungsweg wie die andere therapeutische Substanz verabreicht werden.

Die erfindungsgemäße Zusammensetzung wird vorzugsweise als pharmazeutische Zusammensetzung bereitgestellt und verabreicht. Der Begriff "pharmazeutische Zusammensetzung" umfasst die Wirkstoffkombination Polyacrylsäure und 2-Amino-2-methylpropanol und ein oder mehrere innerte Inhaltstoffe, die als Träger für die Wirkstoffe fungieren.

Polyacrylsäure und 2-Amino-2-methylpropanol sind aus dem Stand der Technik, beispielsweise WO-A-2011/015628 und US-A-2013/0072575 bekannt, nicht hingegen als therapeutisch aktive Wirkstoffkombination im Sinne vorliegender Erfindung.

Die pharmazeutische Zusammensetzung ist vorzugsweise so formuliert, dass sie im Rahmen einer Lokaltherapie oder topischen Therapie angewendet werden kann. Die Wirkstoffe wirken damit nur dort, wo sie benötigt werden, während die infektionsfreien Bereiche geschont werden. Die pharmazeutische Zusammensetzung kann direkt auf die von der Herpes-Infektion befallenen Bereiche aufgetragen werden. Alternativ oder in Ergänzung dazu kann die pharmazeutische Zusammensetzung auch über ein auf den befallenen Bereichen aufliegenden Depot, beispielsweise ein mit der pharmazeutischen Zusammensetzung beschichteten oder getränkten Depotpflaster, verabreicht werden.

Eine Verabreichungsform kann beispielsweise eine Tinktur, ein Aerosol, eine Lösung, Creme, Paste, Lotion, Gel oder Salbe sein. Entsprechende Techniken für die Formulierung und Verabreichung sind aus dem Stand der Technik bekannt, siehe beispielsweise "Remington's Pharmaceutical Sciences" (Mack Publishing Co, Easton Pa.). Im Allgemeinen wird das Mittel von den Patienten 1 bis 20 mal täglich, vorzugsweise mehr als 3 mal täglich, besonders bevorzugt 5 bis 15 mal täglich auf die befallenen Bereiche aufgetragen. Besonders bevorzugt ist ein filmdünner Auftrag. Eine Antrocknung des Films ist empfehlenswert. Zur prophylaktischen Behandlung wird ein mindestens einmaliges wöchentliches Auftragen auf ehemalige Befallstellen empfohlen. Im Behandlungsverlauf wird der Befall im Prodomalstadium verhindert, während bei Anwendung im Erythemstadium das Ziel in der Symptomreduktion und Verhinderung der Bläschenbildung liegt bzw. bei bereits vorhandenen Pappeln, Vesikeln oder Ulcera das Abklingen der Infektion und die schnelle Abheilung der Hautläsionen durch Aufstreichung der erfindungsgemäßen Zusammensetzung erzielt wird.

Der Anteil der aktiven Wirkstoffe Polyacrylsäure und 2-Amino-2-methylpropanol in der pharmazeutischen Zusammensetzung kann variieren und liegt üblicherweise bezogen auf das Gesamtgewicht der Zusammensetzung bei 0,05-15 Gew.-%, vorzugsweise 0,1-5 Gew.-%, weiter vorzugsweise 0,5-2,5 Gew.-%, weiter vorzugsweise 0,75-1,25 Gew.-%, besonders bevorzugt 1 Gew.-% Polyacrylsäure und 0,05-15 Gew.-%, vorzugsweise 0,1-5 Gew.-%, weiter vorzugsweise 0,5-2,5 Gew.-%, weiter vorzugsweise 0,75-1,25 Gew.-%, besonders bevorzugt 1 Gew.-% 2-Amino-2-methylpropanol. Der Wirkstoffanteil ist entsprechend so ausgewählt, dass eine wirksame Dosis erreicht wird.

Die Polyacrylsäure und das 2-Amino-2-methylpropanol kann in "Reinform" oder "gereinigt" verwendet werden, was bedeutet, dass ungewünschte Komponenten, beispielsweise das im Rahmen der Herstellung von Polyacrylsäure verwendete giftige Lösungsmittel Benzol, entfernt wurden.

Polyacrylsäure" (CAS-Nummer 9007-20-9, Merck Index 11, Dezember 1989, ISBN 0-911910-28-X) weist die nachfolgend wiedergegebene Grundstruktur auf 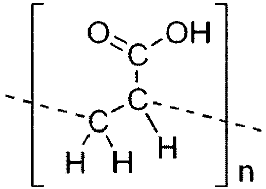 und ist eine synthetisch hergestellte Verbindung und hochmolekulares Polymer der Acrylsäure das in verschiedenen Polymerisationsgraden (Molmasse) vorkommt die alle für die Zwecke vorliegender Erfindung geeignet sind. Beispielhaft genannt seinen die benzolfreien Handelstypen Carbopol 981, Carbopol 971, Carbopol 71G, Carbopol 974P, Carbopol 984, Carbopol 5984, Carbopol 980, Carbopol 934P, Carbopol 941, Carbopol 971, Carbopol 940 oder Carbopol ETD 2001, 2020 und 2050. Die genannten Handelswaren entsprechen den pharmazeutischen Pharmakopöen, s. Ph.Eur. 01/2005:1299. Polyacrylsäure ist gut hautverträglich und auch auf Schleimhäuten anwendbar. Polyacrylsäure wird durch radikalische Polymerisation in Gegenwart von Peroxiden mit Azoverbindungen oder anderen Radikalbildnern als Polymerisationsstartern synthetisiert. Die Herstellung von Polyacrylsäure ist dem Fachmann geläufig und beispielsweise in Bracher et al., Arzneibuch-Kommentar (2010), ISBN 3-8047-2115-X, beschrieben.

2-Amino-2-methylpropanol (CAS-Nummer 124-68-5) weist die nachfolgend wiedergegebene Struktur auf:

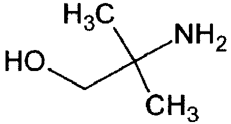

2-Amino-2-methylpropanol kann durch Reduktion der entsprechenden Nitroverbindung gewonnen werden, wie beispielsweise in dem US-Patent 2,174,242 beschrieben.

Gemäß einer bevorzugten Ausführungsform der Erfindung wird die pharmazeutisch aktive Wirkstoffkombination aus Polyacrylsäure und 2-Amino-2-methylpropanol in einer galenischen Zubereitung eingesetzt, die ein oder mehrere pharmazeutische Hilfs- und/oder Zusatzstoffe, vorzugsweise ausgewählt aus der Gruppe bestehend aus einen pharmazeutisch akzeptablen Träger, Füllstoffe, Geruchstoffe und Stabilisatoren enthalten kann. Die Galenik des Medikaments erlaubt vorzugsweise eine kontrollierte und/oder zeitverzögerte Freisetzung der pharmazeutisch aktiven Wirkstoffe. Gemäß einer bevorzugten Ausführungsform der Erfindung umfasst die Zusammensetzung neben der pharmazeutisch aktiven Wirkstoffkombination Polyacrylsäure und 2-Amino-2-methylpropanol die galenischen Hilfsstoffe Ethanol, Glycerin und Wasser, vorzugsweise destilliertes Wasser. Bezogen auf das Gesamtgewicht der Zusammensetzung können enthalten sein
- 20-70 Gew.-%, vorzugsweise 30-60 Gew.-%, weiter vorzugsweise 40-50 Gew.-%, besonders bevorzugt 45 Gew.-% Ethanol,
- 5-45 Gew.-%, vorzugsweise 15-35 Gew.-%, weiter vorzugsweise 20-30 Gew.-%, besonders bevorzugt 25 Gew.-% Glycerin und
- 5-45 Gew.-%, vorzugsweise 15-35 Gew.-%, weiter vorzugsweise 20-30 Gew.-%, besonders bevorzugt 25 Gew.-% Wasser.

Eine Reihe von beispielhaften Kombinationen der Mengen der Einzelkomponenten, wie sie von der erfindungsgemäßen Zusammensetzung umfasst sein können, sind nachfolgend tabellarisch gegeben. Es ist für den Fachmann selbstverständlich, dass sich die einzelnen Komponenten auf maximal 100 aufsummieren können, d.h. in der Praxis nicht von den oberen Endbereichen aller für die Galenik mit verwendeten Komponenten (Ethanol, Glycerin und Wasser) gleichzeitig gebraucht gemacht wird.

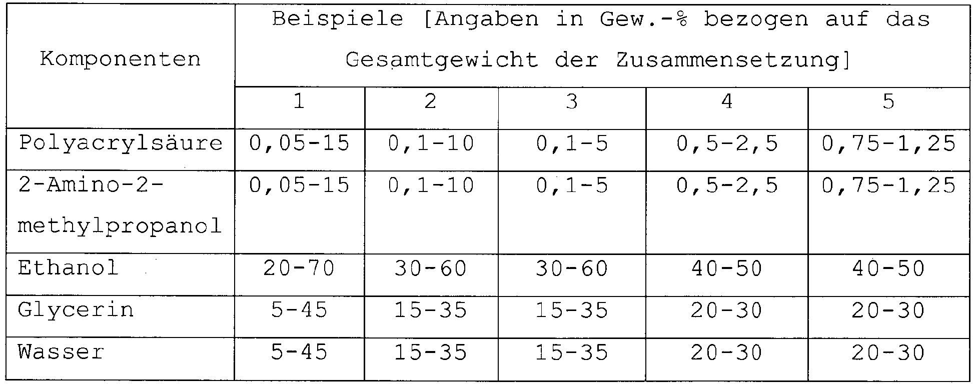

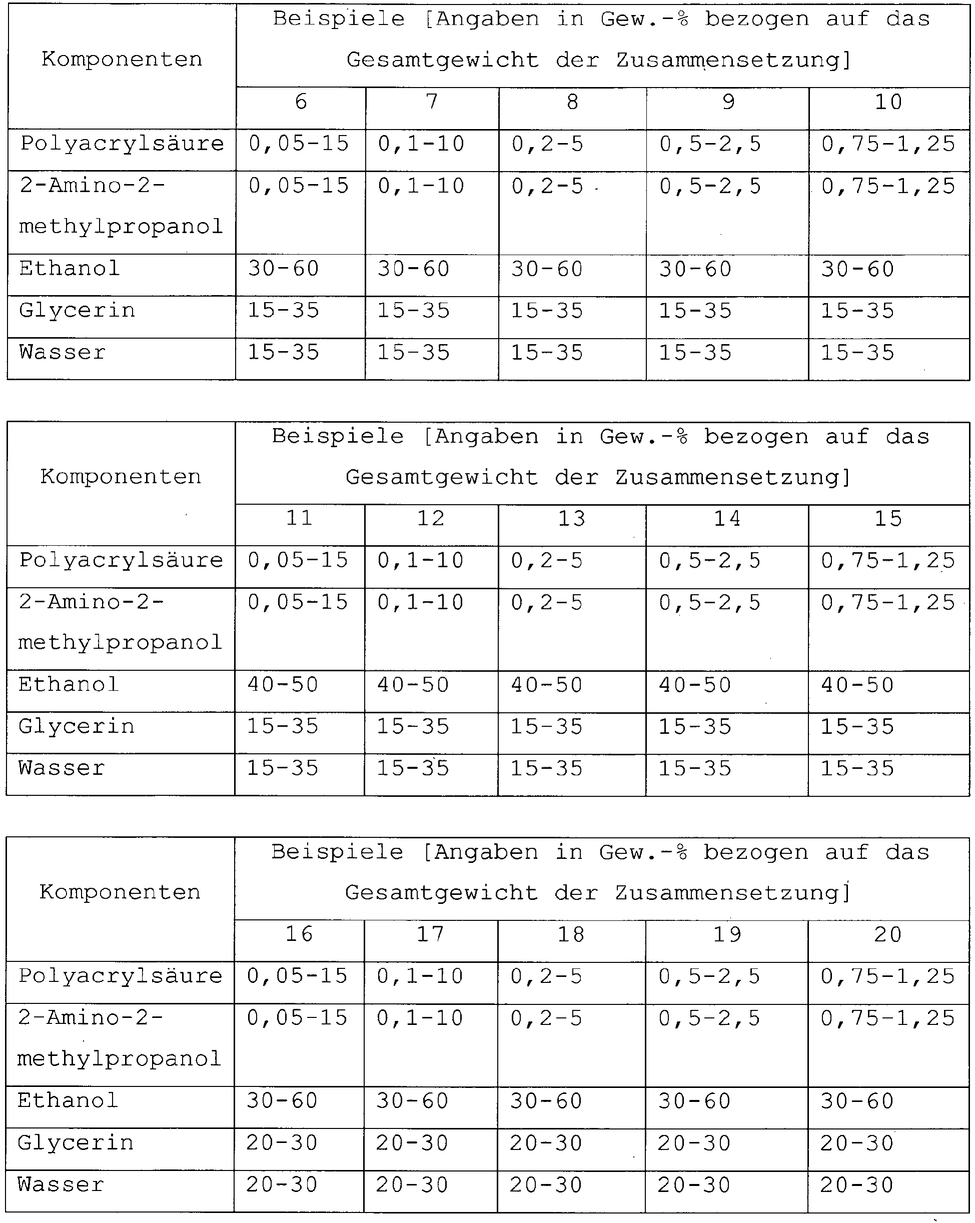

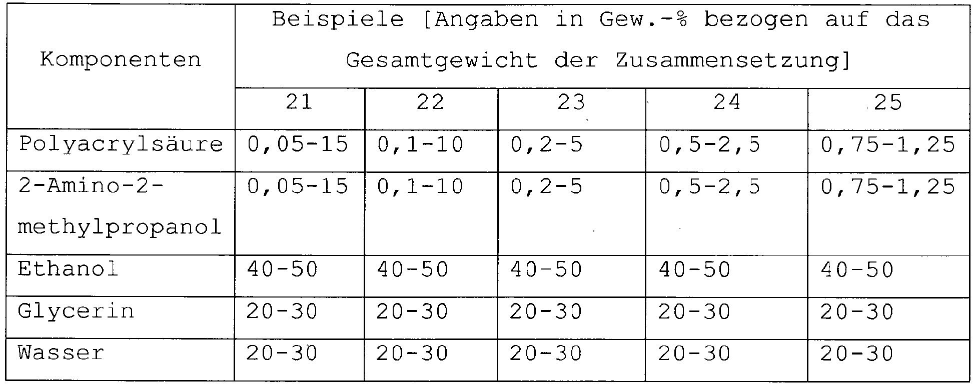

Die Ethanol-Komponente der erfindungsgemäßen Zusammensetzung kann zumindest teilweise mit einem Vergällungsmittel vergällt sein, wobei das Vergällungsmittel vorzugsweise ausgewählt ist aus der Gruppe bestehend aus Propanon, Butanon, Denatoniumbenzoat, Minzöl, Petrolether, Toluol, Cyclohexan, Phthalsäurediethylester und Pyridin.

Die erfindungsgemäße Zusammensetzung kann des weiteren penetrationsfördernde Hilfstoffe, wie z.B. Propylenglycol oder Dimethylsulfoxid umfassen.

Besonders bevorzugt ist es im Rahmen der Erfindung, wenn der pH-Wert der für die Anwendung fertigen Zusammensetzung in wässriger Lösung 7-9, vorzugsweise 7,5-8,5, weiter vorzugsweise 7,75-8,25 und besonders bevorzugt 8 beträgt (Messung nach DIN ISO 10390:2005-12). Die Herstellung der erfindungemäßen Zusammensetzung erfolgt unter sterilen Bedingungen. Dabei werden bekannte und eingeführte Verfahren (Mischen, Rühren, Homogenisieren und Filtrieren) mit ebenfalls üblichen Geräten angewendet (s. z.B. Remington's Pharmaceutical Sciences und U.S. Pharmacopeia und Viogt, Rudolf, Pharmazeutische Technologie für Studium und Beruf, 7. Auflage, Berlin (1993). Im Rahmen der Herstellung kann das Vermischen der Bestandteile vorzugsweise durch Rühren geschehen. Bevorzugte Zeiträume für das Vermischen sind 1 bis 20h.

Die erfindungsgemäße Zusammensetzung kann als Einzel- oder Mehrfachdosis verpackt sein. Die bestmögliche Dosierung für den einzelnen Patienten wird bestimmt von z.B. dem Allgemeinzustand des Patienten, Krankheitsfortschritt und der Applikationsform. Bei Personen, deren Herpes sehr häufig, üblicherweise mehr als sechsmal pro Jahr, rezidiviert und deshalb in ihrem Wohlbefinden stark beeinträchtigt sind, kommt eine prophylaktische Langzeit-Suppresionstherapie mit der erfindungsgemäßen Zusammensetzung in Betracht, die dem Patienten vorzugsweise in einer Mehrfachdosisverpackung zur Selbstmedikation Verfügung gestellt wird.

Die Erfindung soll nun im Folgenden weiter in den Beispielen beschrieben werden, ohne auf diese beschränkt zu sein.

### Beispiele

### Beispiel 1

Die nachfolgend genannte erfindungsgemäße Formulierung mit einem schwach alkalischen pH-Wert von 7,8 wurde unter Reinraumbedingungen unter zweistündigen mischen der Komponenten bei 20°C hergestellt. Die gelige Lösung und in sterile Behältnisse abgefüllt um am Patienten später mit Pinsel, Spatel oder Finger auf die Haut aufgetragen zu werden (s. Beispiel 2).

| | |
|---|---|
| Polyacrylsäure (CAS-Nr. 9007-20-9) | 1 Gew.-% |
| 2-Amino-2-methylpropanol (CAS-Nr. 124-68-5) | 1 Gew.-% |
| Alkohol denat. (CAS-Nr. 64-17-5) | ca. 45 Gew.-% |
| Glycerin (CAS-Nr. 56-81-5) | ca. 25 Gew.-% |
| Aqua dest. (CAS-Nr. 7732-18-5) | ca. 25 Gew.-% |

### Beispiel 2

Die nachfolgenden Anwendungsberichte basieren auf ärztlichen Fallbeispielen (Fälle 1-3) sowie Eigenerfahrungen von Patienten denen die erfindungsgemäße Zusammensetzung gemäß Beispiel 1 zur Selbstmedikation überlassen wurde (Fälle 4-8).

| **Fall 1:** | |
|---|---|
| ♀, 31 Jahre alt, | |
| Diagnose: | Herpes labialis chronisch |
| | glgt. Herpes genitalis |
| 1. Visite 15.03.2013 | |
| | Typischer Lippenbefall in Pappelphase |
| | Typischer linkslateraler Befall der Labia minora |
| | Anleitung zur Behandlung |
| 2. Visite 18.03.2013 | |
| | Lippe größtenteils nur noch schwach rot |
| | Genitalbefall flächig in Abheilung |
| 3. Visite 22.03.2013 | |
| | Keine Symptome mehr nachweisbar |

| **Fall 2:** | |
|---|---|
| ♂, 21 Jahre alt, | |
| Diagnose: | Herpes labialis nach 4. Schub im ersten Jahr |
| 1. Visite 22.03.2013 | |
| | Erythemphase |
| | Anleitung zur Behandlung |
| 2. Visite 25.03.2013 | |
| | Keine Symptome |

| **Fall 3:** | |
|---|---|
| ♀, 51 Jahre alt, | |
| Diagnose: | Zustand nach Tuberkulosebehandlung vor 2 Jahren Jetzt ca. 6. Schub eines Herpes labialis |
| 1. Visite 03.04.2013 | |
| | Prodomalstadium Lippe mit Kribbeln in ehemaligem |
| | Hautareal rechts unten |
| | Anleitung zur Behandlung |
| 2. Visite 06.04.2013 | |
| | Keine Symptome mehr nachweisbar |

| **Fall 4:** | |
|---|---|
| Versuchsperson | |
| Geschlecht: | männlich |
| Alter: | 42 |
| | |

| Erkrankung | |
|---|---|
| Bezeichnung: | herpes labialis (Lippenherpes) |
| Stadium/Symptome: | erste Bläschenbildung; Juckreiz/Entzündungsschmerz |
| | |

| Anwendung | |
|---|---|
| Form: | äußerliche Anwendung unmittelbar auf die erkrankte Hautstelle |
| Häufigkeit: | mindestens 10 Mal täglich |

| Heilungsverlauf | |
|---|---|
| Kein weiteres Bläschenwachstum; Rückgang des Juckreizes/Entzündungsschmerzes; Bläschen trocknete binnen weniger Stunden aus; sämtliche Symptome waren nach drei Tagen verschwunden | |

| **Fall 5:** | |
|---|---|
| Versuchsperson | |
| Geschlecht: | männlich |
| Alter: | 42 |
| | |

| Erkrankung | |
|---|---|
| Bezeichnung: | herpes labialis (Lippenherpes) |
| Stadium/Symptome: | erste Rötung und punktueller Juckreiz/Entzündungsschmerz; noch keine Bläschenbildung |
| | |

| Anwendung | |
|---|---|
| Form: | äußerliche Anwendung unmittelbar auf die erkrankte Hautstelle |
| Häufigkeit: | mindestens 10 Mal täglich |
| | |
| Heilungsverlauf | |
| Keine Bläschenbildung; Rückgang des Juckreizes/Entzündungsschmerz; sämtliche Symptome waren nach einem Tag verschwunden | |

| **Fall 6:** | |
|---|---|
| | |
| Versuchsperson | |
| Geschlecht: | weiblich |
| Alter: | 39 |
| | |

| Erkrankung | |
|---|---|
| Bezeichnung: | herpes labialis (Lippenherpes) |
| Stadium/Symptome: | erste Bläschenbildung; Juckreiz/Entzündungsschmerz |
| | |

| Anwendung | |
|---|---|
| Form: | äußerliche Anwendung unmittelbar auf die erkrankte Hautstelle |
| Häufigkeit: | mindestens 10 Mal täglich |
| | |

| Heilungsverlauf | |
|---|---|
| Kein weiteres Bläschenwachstum; Rückgang des Juckreizes/Entzündungsschmerzes; Bläschen trocknete binnen weniger Stunden aus; sämtliche Symptome waren nach drei Tagen verschwunden | |

| **Fall 7:** | |
|---|---|
| | |
| Versuchsperson | |
| Geschlecht: | weiblich |
| Alter: | 40 |
| | |

| Erkrankung | |
|---|---|
| Bezeichnung: | herpes labialis (Lippenherpes) |
| Stadium/Symptome: | fortgeschrittene Blasenbildung; Juckreiz/Entzündungsschmerz |
| | |

| Anwendung | |
|---|---|
| Form: | äußerliche Anwendung unmittelbar auf die erkrankte Hautstelle |
| Häufigkeit: | mindestens 10 Mal täglich |
| | |

| Heilungsverlauf | |
|---|---|
| Kein weiteres Bläschenwachstum; Rückgang des Juckreizes/Entzündungsschmerzes; Bläschen trocknete binnen weniger Stunden aus; sämtliche Symptome waren nach drei Tagen verschwunden | |

| **Fall 8:** | |
|---|---|
| | |
| Versuchsperson | |
| Geschlecht: | weiblich |
| Alter: | 32 |
| | |

| Erkrankung | |
|---|---|
| Bezeichnung: | herpes labialis (Lippenherpes) |
| Stadium/Symptome: | erste Bläschenbildung; Juckreiz/Entzündungsschmerz |
| | |

| Anwendung | |
|---|---|
| Form: | äußerliche Anwendung unmittelbar auf die erkrankte Hautstelle |
| Häufigkeit: | mindestens 10 Mal täglich |
| | |

| Heilungsverlauf | |
|---|---|
| Kein weiteres Bläschenwachstum; Rückgang des Juckreizes/Entzündungsschmerzes; Bläschen trocknete binnen weniger Stunden aus; sämtliche Symptome waren nach drei/vier Tagen verschwunden | |

Es konnte festgestellt werden, dass durch die Verabreichung der erfindungemäßen Zusammensetzung gemäß Beispiel 1 eine deutliche Verringerung der Dauer der Ausbrüche erzielt werden konnte. Die Dauer des vesikularen Stadiums verkürzte sich, die Läsionen heilten schneller ab und die Schmerzen, Juckreiz und Brennen wurden günstig beeinflusst. Gegenanzeigen oder Nebenwirkungen traten keine auf. Vergleicht man die Resultate bei frühem Therapiebeginn (Prodromal- oder Erythemstadium) mit denjenigen bei spätem Therapiebeginn (Pappel- und Bläschen-Stadium), so lässt sich daraus schließen, dass die Abheilung unabhängig vom Stadium zum Zeitpunkt des Therapiebeginns beschleunigt wurde. Auch bei späten Applikationen in der Pappel- und Bläschenphase zeigte die erfindungsgemäße Zusammensetzung eine signifikante Wirkung auf die Abheilungszeit der Läsionen. Die Beispiele zeigen also einen vom Behandlungsbeginn weitgehend unabhängigen therapeutischen Effekt.

## Patentansprüche

1. Zusammensetzung umfassend
a) Polyacrylsäure und
b) 2-Amino-2-methylpropanol
als therapeutische Wirkstoffe zur Verwendung als Medikament.

2. Zusammensetzung umfassend
a) Polyacrylsäure und
b) 2-Amino-2-methylpropanol
als therapeutische Wirkstoffe zur Verwendung bei der prophylaktischen und/oder therapeutischen Behandlung von Herpes-Infektionen.

3. Zusammensetzung zur Verwendung nach Anspruch 2, **dadurch gekennzeichnet, dass** die zu behandelnden Herpes-Infektion *Herpes simplex labialis* (Lippenherpes), *Gingivostomatitis herpetica* (Mundfäule) oder *Herpes simplex genitalis* (Genitalherpes) sind.

4. Zusammensetzung zur Verwendung nach Anspruch 2, **dadurch gekennzeichnet, dass** die zu behandelnde Herpes-Infektion eine Infektion mit einem Virus ist, ausgewählt ist aus der Gruppe bestehend aus
- Herpes-simplex-Virus Typ 1,
- Herpes-simplex-Virus Typ 2 und
- Varicella-Zoster-Virus (Herpes zoster).

5. Zusammensetzung zur Verwendung nach einem der vorangehenden Ansprüche, **dadurch gekennzeichnet, dass** die Zusammensetzung eine therapeutisch wirksame Menge an Polyacrylsäure und 2-Amino-2-methylpropanol umfasst.

6. Zusammensetzung zur Verwendung nach einem der vorangehenden Ansprüche, **dadurch gekennzeichnet, dass** die Zusammensetzung bezogen auf das Gesamtgewicht der Zusammensetzung enthält:
a) 0,05-15 Gew.-%, vorzugsweise 0,1-5 Gew.-%, weiter vorzugsweise 0,5-2,5 Gew.-%, weiter vorzugsweise 0,75-1,25 Gew.-%, besonders bevorzugt 1 Gew.-% Polyacrylsäure und
b) 0,05-15 Gew.-%, vorzugsweise 0,1-5 Gew.-%, weiter vorzugsweise 0,5-2,5 Gew.-%, weiter vorzugsweise 0,75-1,25 Gew.-%, besonders bevorzugt 1 Gew.-% 2-Amino-2-methylpropanol.

7. Zusammensetzung zur Verwendung nach einem der vorangehenden Ansprüche, des weiteren umfassend ein oder mehrere pharmazeutische Hilfs- und/oder Zusatzstoffe, vorzugsweise ausgewählt aus der Gruppe bestehend aus einem pharmazeutisch akzeptablen Träger, Füllstoffe, Geruchsstoffe und Stabilisatoren.

8. Zusammensetzung zur Verwendung nach einem der vorangehenden Ansprüche umfassend
c) Ethanol,
d) Glycerin und
e) Wasser.

9. Zusammensetzung zur Verwendung nach Anspruch 8, **dadurch gekennzeichnet, dass** die Zusammensetzung bezogen auf das Gesamtgewicht der Zusammensetzung enthält:
c) 20-70 Gew.-%, vorzugsweise 30-60 Gew.-%, weiter vorzugsweise 40-50 Gew.-%, besonders bevorzugt 45 Gew.-% Ethanol,
d) 5-45 Gew.-%, vorzugsweise 15-35 Gew.-%, weiter vorzugsweise 20-30 Gew.-%, besonders bevorzugt 25 Gew.-% Glycerin und
e) 5-45 Gew.-%, vorzugsweise 15-35 Gew.-%, weiter vorzugsweise 20-30 Gew.-%, besonders bevorzugt 25 Gew.-% Wasser.

10. Zusammensetzung zur Verwendung nach Anspruch 8 oder 9, **dadurch gekennzeichnet, dass** die Komponente c) zumindest teilweise mit einem Vergällungsmittel vergällt ist, wobei das Vergällungsmittel vorzugsweise ausgewählt ist aus der Gruppe bestehend aus Propanon, Butanon, Denatoniumbenzoat, Minzöl, Petrolether, Toluol, Cyclohexan, Phthalsäurediethylester und Pyridin.

11. Zusammensetzung zur Verwendung nach einem der vorangehenden Ansprüche, **dadurch gekennzeichnet, dass** die Zusammensetzung zur topischen Verabreichung formuliert ist.

12. Zusammensetzung zur Verwendung nach einem der vorangehenden Ansprüche, **dadurch gekennzeichnet, dass** die Verabreichungsform ausgewählt ist aus der Gruppe bestehend aus Tinktur, Gel, Paste, Lotion, Salbe, Creme oder Aerosol.

13. Zusammensetzung zur Verwendung nach einem der vorangehenden Ansprüche, **dadurch gekennzeichnet, dass** die Zusammensetzung in wässriger Lösung einen pH-Wert von 7-9, vorzugsweise 7,5-8,5, weiter vorzugsweise 7,75-8,25, besonders bevorzugt 8 aufweist.

14. Zusammensetzung zur Verwendung nach einem der vorangehenden Ansprüche, **dadurch gekennzeichnet, dass** die Zusammensetzung als Einzel- oder Mehrfachdosis verpackt ist.

15. Zusammensetzung zur Verwendung nach einem der vorangehenden Ansprüche, **dadurch gekennzeichnet, dass** die Zusammensetzung nur Polyacrylsäure und 2-Amino-2-methylpropanol als therapeutisch aktive Wirkstoffkombination umfasst.

## Claims

1. Composition comprising
a) polyacrylic acid and
b) 2-amino-2-methyl-1-propanol
as therapeutic agents for use as medication.

2. Composition comprising
a) polyacrylic acid and
b) 2-amino-2-methyl-1-propanol
as therapeutic agents for use in the prophylactic and/or therapeutic treatment of herpes infections.

3. Composition for use according to claim 2, **characterised in that** the herpes infections to be treated are *Herpes simplex labialis* (oral herpes), *Gingivostomatitis herpetica* (orolabial herpes) or *Herpes simplex genitalis* (genital herpes).

4. Composition for use according to claim 2, **characterised in that** the herpes infection to be treated is an infection by a virus selected from the group consisting of:
- Herpes simplex virus type 1,
- Herpes simplex virus type 2 and
- varicella-zoster virus (herpes zoster).

5. Composition for use according to any of the preceding claims, **characterised in that** the composition comprises a therapeutically effective amount of polyacrylic acid and 2-amino-2-methyl-1-propanol.

6. Composition for use according to any of the preceding claims, **characterised in that** the composition contains, based on the total weight of the composition:
a) 0.05-15 wt.%, preferably 0.1-5 wt.%, more preferably 0.5-2.5 wt.%, more preferably 0.75-1.25 wt.%, most preferably 1 wt.% polyacrylic acid and
b) 0.05-15 wt.%, preferably 0.1-5 wt.%, more preferably 0.5-2.5 wt.%, more preferably 0.75-1.25 wt.%, most preferably 1 wt.% 2-amino-2-methyl-1-propanol.

7. Composition for use according to any of the preceding claims, further comprising one or more pharmaceutical excipients and/or additives, preferably selected from the group consisting of a pharmaceutically acceptable carrier, fillers, odorants and stabilisers.

8. Composition for use according to any of the preceding claims, comprising
c) ethanol
d) glycerol and
e) water.

9. Composition for use according to claim 8, **characterised in that** the composition contains, based on the total weight of the composition:
c) 20-70 wt.%, preferably 30-60 wt.%, more preferably 40-50 wt.%, most preferably 45 wt.% ethanol,
d) 5-45 wt.%, preferably 15-35 wt.%, more preferably 20-30 wt.%, most preferably 25 wt.% glycerol and
e) 5-45 wt.%, preferably 15-35 wt.%, more preferably 20-30 wt.%, most preferably 25 wt.% water.

10. Composition for use according to either claim 8 or claim 9, **characterised in that** the component c) is at least partially denatured by means of a denaturing agent, the denaturing agent preferably being selected from the group consisting of propanone, butanone, denatonium benzoate, mint oil, petroleum ether, toluene, cyclohexane, diethyl phthalate and pyridine.

11. Composition for use according to any of the preceding claims, **characterised in that** the composition is formulated to be administered topically.

12. Composition for use according to any of the preceding claims, **characterised in that** the form of administration is selected from the group comprising tincture, gel, paste, lotion, ointment, cream or aerosol.

13. Composition for use according to any of the preceding claims, **characterised in that**, in aqueous solution, the composition has a pH of 7-9, preferably 7.5-8.5, more preferably 7.75-8.25, most preferably 8.

14. Composition for use according to any of the preceding claims, **characterised in that** the composition is packaged as a single dose or multiple dose.

15. Composition for use according to any of the preceding claims **characterised in that** the composition contains only polyacrylic acid and 2-amino-2-methyl-1-propanol as a therapeutically active combination of substances.

## Revendications

1. Composition comprenant
a) un poly(acide acrylique), et
b) du 2-amino-2-méthylpropanol,
en tant que principes actifs pour utilisation en tant que médicament.

2. Composition comprenant
a) un poly(acide acrylique), et
b) du 2-amino-2-méthylpropanol,
en tant que principes actifs thérapeutiques pour utilisation lors du traitement prophylactique et/ou thérapeutique d'infections de type herpès.

3. Composition pour l'utilisation selon la revendication 2, **caractérisée en ce que** l'infection de type herpes devant être traitée est Herpes simplex labialis (herpès labial), Gingivostomatitis herpetica (gingivo-stomatite herpétique) ou Herpes simplex genitalis (herpès simplex génital).

4. Composition pour l'utilisation selon la revendication 2, **caractérisée en ce que** l'infection de type herpès devant être traitée est une infection par un virus choisi dans le groupe consistant en
- le virus herpès simplex de type 1,
- le virus herpès simplex de type 2, et
- le virus de la varicelle et du zona (Herpes zoster).

5. Composition pour l'utilisation selon l'une des revendications précédentes, **caractérisée en ce que** la composition comprend une quantité thérapeutiquement efficace d'un poly(acide acrylique) et de 2-amino-2-méthylpropanol.

6. Composition pour l'utilisation selon l'une des revendications précédentes, **caractérisée en ce que** la composition contient, par rapport au poids total de la composition :
a) 0,05-15 % en poids, de préférence 0,1-5 % en poids, d'une manière plus préférée 0,5-2,5 % en poids, d'une manière plus préférée 0,75-1,25 % en poids, d'une manière particulièrement préférée 1 % en poids de poly(acide acrylique), et
b) 0,05-15 % en poids, de préférence 0,1-5 % en poids, d'une manière plus préférée 0,5-2,5 % en poids, d'une manière plus préférée 0,75-1,25 % en poids, d'une manière particulièrement préférée 1 % en poids de 2-amino-2-méthylpropanol.

7. Composition pour l'utilisation selon l'une des revendications précédentes, qui comprend en outre un ou plusieurs adjuvants et/ou additifs pharmaceutiques, choisis de préférence dans le groupe consistant en un support, des charges, des arômes et des stabilisants pharmaceutiquement acceptables.

8. Composition pour l'utilisation selon l'une des revendications précédentes, comprenant
c) de l'éthanol,
d) du glycérol, et
e) de l'eau.

9. Composition pour l'utilisation selon la revendication 8, **caractérisée en ce que** la composition contient, par rapport au poids total de la composition :
c) 20-70 % en poids, de préférence 30-60 % en poids, d'une manière plus préférée 40-50 % en poids, d'une manière particulièrement préférée 45 % en poids d'éthanol,
d) 5-45 % en poids, de préférence 15-35 % en poids, d'une manière plus préférée 20-30 % en poids, d'une manière particulièrement préférée 25 % en poids de glycérol, et
e) 5-45 % en poids, de préférence 15-35 % en poids, d'une manière plus préférée 20-30 % en poids, d'une manière particulièrement préférée 25 % en poids d'eau.

10. Composition pour l'utilisation selon la revendication 8 ou 9, **caractérisée en ce que** le composant c) est au moins partiellement dénaturé par un agent dénaturant, l'agent dénaturant étant de préférence choisi dans le groupe consistant en la propanone, la butanone, le benzoate de dénatonium, l'essence de menthe, l'éther de pétrole, le toluène, le cyclohexane, l'ester diéthylique de l'acide phtalique et la pyridine.

11. Composition pour l'utilisation selon l'une des revendications précédentes, **caractérisée en ce que** la composition est formulée pour administration topique.

12. Composition pour l'utilisation selon l'une des revendications précédentes, **caractérisée en ce que** la forme galénique est choisie dans le groupe consistant en une teinture, un gel, une pâte, une lotion, une pommade, une crème ou un aérosol.

13. Composition pour l'utilisation selon l'une des revendications précédentes, **caractérisée en ce que** la composition présente en solution aqueuse un pH de 7-9, de préférence de 7,5-8,5, d'une manière plus préférée de 7,75-8,25, d'une manière particulièrement préférée de 8.

14. Composition pour l'utilisation selon l'une des revendications précédentes, **caractérisée en ce que** la composition est conditionnée en monodoses ou multidoses.

15. Composition pour l'utilisation selon l'une des revendications précédentes, **caractérisée en ce que** la composition ne comprend, en tant qu'association de principes actifs thérapeutiquement actifs, qu'un poly(acide acrylique) et du 2-amino-2-méthylpropanol.
